(19) Europäisches Patentamt
European Patent Office
Office européen des brevets

(11) **EP 3 943 096 A1**

(12) **EUROPEAN PATENT APPLICATION**

(43) Date of publication:
**26.01.2022 Bulletin 2022/04**

(21) Application number: **21186828.6**

(22) Date of filing: **20.07.2021**

(51) International Patent Classification (IPC):
**A61K 36/9066** (2006.01)     **B01D 11/00** (2006.01)

(52) Cooperative Patent Classification (CPC):
**B01D 11/0288; A61K 36/9066; B01D 11/028;**
A61K 2236/00; B01D 9/0054

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.07.2020 IN 202041031859**

(71) Applicant: **Star Hi Herbs Pvt Ltd.
Bangaluru, Rural. Karnataka 560105 (IN)**

(72) Inventors:
• **Hirehal, Hussain Mirza Firoz
560105 Bengaluru (IN)**
• **Nanjundaiah, Singam Setty
560105 Bengaluru (IN)**

(74) Representative: **Dargiewicz, Joanna
JD&P Patent Attorneys
Joanna Dargiewicz & Partners
Ul. Mysliborska 93A/50
03-185 Warszawa (PL)**

(54) **METHOD OF SYNTHESIZING A TURMERIC BASED COMPOSITION HAVING ENHANCED BIOAVAILABILITY**

(57)     The present invention relates to a method of synthesizing a turmeric based composition wherein the composition has an enhanced bioavailability in blood. The composition is prepared by micronization and homogenization of the turmeric extract. The composition is a water dispersible liquid.

```
┌─────────────────────────────────────┐
│  Preparing an extract of turmeric    │── 101
└─────────────────────────────────────┘
                   │
                   ▼
┌─────────────────────────────────────┐
│      Micronizing the extract         │── 102
└─────────────────────────────────────┘
                   │
                   ▼
┌─────────────────────────────────────┐
│  Dispersing the micronized extract in│── 103
│        water at a high rpm           │
└─────────────────────────────────────┘
                   │
                   ▼
┌─────────────────────────────────────┐
│     Homogenizing the extract         │── 104
└─────────────────────────────────────┘
                   │
                   ▼
┌─────────────────────────────────────┐
│      Spray drying the extract        │── 105
└─────────────────────────────────────┘
```

**FIG. 1**

EP 3 943 096 A1

**Description**

**BACKGROUND**

*Technical Field of Invention*

The present invention generally relates to phytoconstituent based compositions and particularly to turmeric based herbal composition. The present invention more particularly relates to a method of synthesizing a turmeric composition having enhanced bioavailability, anticancer, antioxidant, anti-inflammatory, hepatoprotective and cognitive properties.

*Description of Related Art*

Curcumin [1,7-bis(4-hydroxy-3-methoxyphenyl)-1,6-heptadiene-3,5-dione] is the major yellow pigment of turmeric, a commonly used spice, derived from the rhizome of the herb Curcuma longa Linn. In the Indian subcontinent and Southeast Asia, turmeric has traditionally been used as a treatment for inflammation, skin wounds, and tumors. Curcumin has antibacterial and antifungal, anti-inflammatory, anti-allergic and wound healing properties. Curcumin is currently marketed as a dietary supplement in several countries (United States, India, Japan, Korea, Thailand, China, Turkey, South Africa, Nepal, and Pakistan). Nevertheless, the use of curcumin has been limited due to its poor aqueous solubility, chemical instability, photo degradation, rapid metabolism, and short half-life. Moreover, orally administered curcumin shows poor bioavailability.

Currently in market maximum strength of water dispersible curcuminoids available are up to 30%. Curcumin tends to be rapidly metabolized and is not easily absorbed into the blood stream, which limits its bioactive properties.

Many formulations of curcumin have been developed and are available in the market. Most of the formulations relied on the use of piperine, a component responsible for the pungency of black pepper and long pepper as an adjuvant due to its ability to inhibit the hepatic and intestinal metabolism of curcumin. However, the use of piperine to increase the absorption rate of curcumin was not successful. Preventing the metabolism alone without enhancing the solubility does not contribute much to the improved bioavailability. The use of other adjuvants such as green tea or soya bean extracts was also not found to be good enough in increasing the bioavailability of curcumin.

Hence, there is a need to develop a novel process to produce curcuminoids of higher strength than available grades in market to minimize the dose/quantity and make more bioavailable even without carriers/emulsifiers. Further there is also a need to provide a method of synthesizing a turmeric composition having enhanced bioavailability, anticancer, antioxidant, anti-inflammatory, hepatoprotective and cognitive effects.

The value additions and above-mentioned shortcomings, disadvantages and problems are addressed herein, as detailed below.

**SUMMARY OF THE INVENTION**

The primary object of the present invention is to provide a method of synthesizing a composition having turmeric.

Another object of the present invention is to provide a method of synthesizing a water dispersible composition containing curcuminoids.

Yet another object of the present invention is to provide a method of synthesizing a water dispersible composition containing curcuminoids and having enhanced bioavailability, anticancer, antioxidant, anti-inflammatory, hepatoprotective and cognitive effects.

Yet another object of the present invention is to provide a method of synthesizing a water dispersible composition containing curcuminoids having increased absorption, rapid metabolism and rapid systemic elimination.

Yet another object of the present invention is to provide a method of synthesizing a water dispersible composition containing curcuminoids having increased solubility.

Yet another object of the present invention is to provide a method of preparation of phytoconstituent curcumin from turmeric powder with enhanced bioavailability and to augment the biological activity of curcumin.

The embodiments of the present invention provide a method of synthesizing a turmeric based composition comprising preparing an extract of turmeric, micronizing the extract, dispersing the micronized extract in water at a high rpm, homogenizing the extract, and spray drying the extract.

According an embodiment of the present invention, the turmeric extract is 95%.

According an embodiment of the present invention, the micronization is performed at an input pressure of 1 to 7 bars and an injector pressure of 0.2 to 0.5 bar using a mcironizer machine.

According an embodiment of the present invention, the micronization is performed at a loading rate of 0.1 to 5Kg/hr keeping gas flow rate of 30 to 100 m/hr of the micronizer machine.

According an embodiment of the present invention, the dispersion is done in water at 5000 to 7500 rpm using high shear blades at hot condition temperature of 70-80°C.

According an embodiment of the present invention, the extract is spray dried at an inlet temperature of 190-200°C and outlet temperature of 90-100°C at a feed rate of 60L/hr.

According to an embodiment of the present invention, the micronization is done for 20 hour for a batch of 100 kg.

According to an embodiment of the present invention, the turmeric based composition has an enhanced bioavailability, antioxidant, antiproliferative and/or anti-inflammatory useful for treatment of cancer and inflammation.

According to an embodiment of the present invention, the turmeric based composition has a therapeutically sufficient amount of the water dispersible curcumin complex of turmeric as active ingredients and a pharmaceutically acceptable vehicle or carrier.

According to an embodiment of the present invention, the therapeutically sufficient amount is between 0.01 mg and 1000 mg per kilogram of body weight of the individual to which it is administered.

According to an embodiment of the present invention, a novel water dispersible curcumin having enhanced bioavailability is provided. The composition is used for making of effervescent beverages, gummies, soft gels, tablets, capsule, shots, drinks.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the spirit thereof, and the embodiments herein include all such modifications.

## BRIEF DESCRIPTION OF DRAWINGS

The other objects, features and advantages will occur to those skilled in the art from the following description of the preferred embodiment and the accompanying drawings in which:

FIG. 1 is a flow chart showing the steps involved in the method of synthesizing a turmeric based composition, according to an embodiment of the present invention.

FIG. 1A is a flow chart showing the steps involved in the process of preparing an extract of turmeric, according to an embodiment of the present invention.

FIG. 2 shows the plasma concentration of curcuminoids after application of a single oral dose(500mg/kg.b.w) of Turmimax® in rats, according to an embodiment of the present invention.

FIG. 3 showings the DPPH free radical scavenging activity (IC50$\mu$g/ml) of Turmimax®/composition of the present invention, according to an embodiment of the present invention.

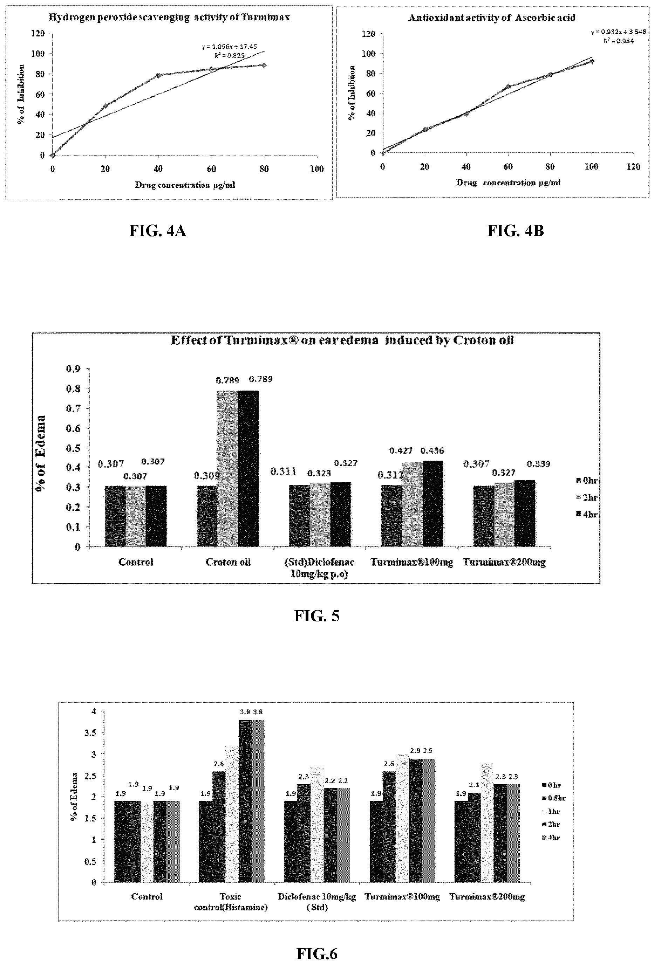

FIG-4A shows the Hydrogen peroxide scavenging activity (IC50$\mu$g/ml) of Turmimax®/composition of the present invention, and FIG. 4B shows the antioxidant activity of the ascorbic acid, according to an embodiment of the present invention.

FIG. 5 shows the anti-inflammatory activity of Turmimax®/composition of the present invention on croton oil induced ear edema in mice, according to an embodiment of the present invention.

FIG. 6 shows the anti-inflammatory activity of Turmimax®/composition on histamine-induced paw edema in rats, according to an embodiment of the present invention.

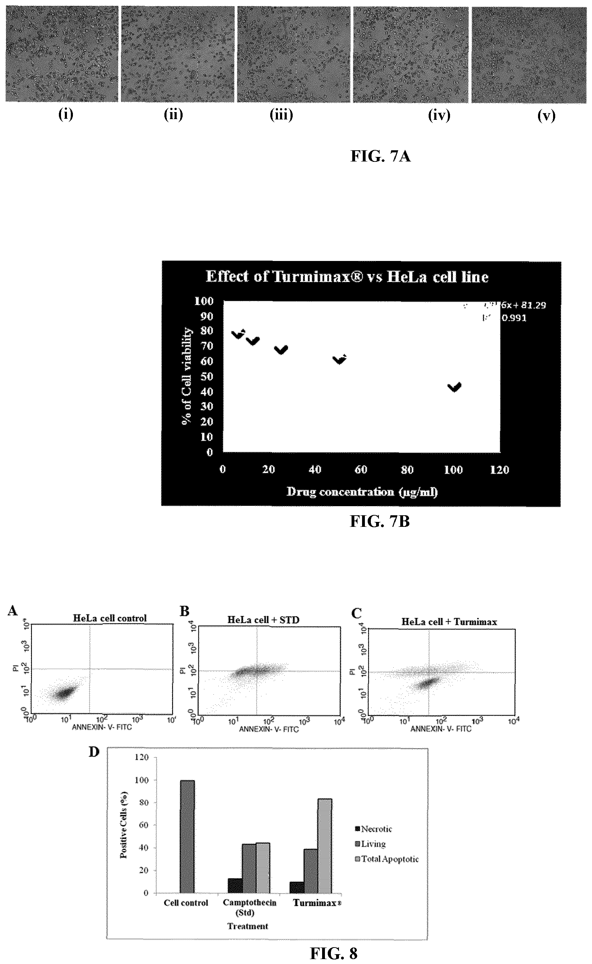

FIG. 7A shows the morphological changes to different concentrations of Turmimax® for 24 hrs in Hela cells and FIG. 7B shows cytotoxicity effect of Turmimax® against HeLa cell line after 24 h incubation.

FIG. 8 shows the Apoptosis Induction of Turmimax® in HeLa cells, according to an embodiment of the present invention.

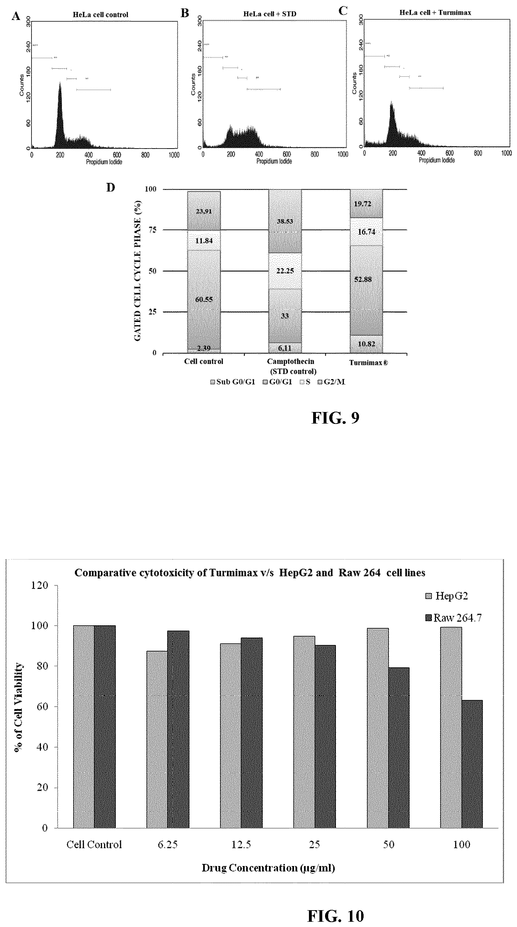

FIG. 9 shows the Cell Cycle Analysis of Turmimax® against HeLa cells using BD FACS Caliber, according to an embodiment of the present invention.

FIG. 10 shows the percentage viability of HepG2 and Raw 264.7 cells treated with various concentrations of Turmimax® for 24 hrs as determined by MTT assay, according to an embodiment of the present invention.

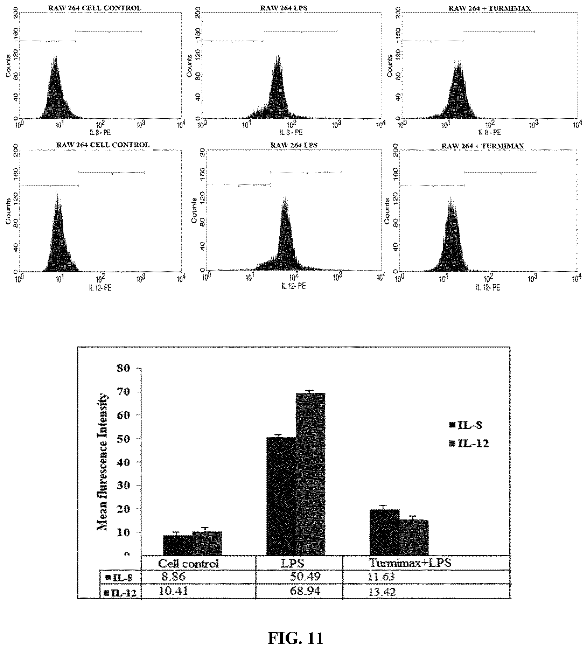

FIG. 11 shows the Flow cytometry histograms of mean pro-inflammatory cytokines (IL-8 & IL-12) in Raw 264.7 cells pre-stimulated with LPS followed by 24 hrs exposure to Turmimax®, according to an embodiment of the present invention.

## DETAILED DESCRIPTION OF EMBODIMENTS

In the following detailed description, a reference is made to the accompanying drawings that form a part hereof, and in which the specific embodiments that may be practiced is shown by way of illustration. The embodiments are described in sufficient detail to enable those skilled in the art to practice the embodiments and it is to be understood that the logical, mechanical, electronic and other changes may be made without departing from the scope of the embodiments. The following detailed description is therefore not to be taken in a limiting sense.

The various embodiments of the present invention provide a water dispersible solution of turmeric which overcomes the drawback of poor bioavailability exhibited by currently available curcumin compositions. The composition consists of curcumin as the major ingredient with enhanced bioavailability by increasing solubility of curcumin and augments the utmost utilization of curcumin for its diverse health benefits. The present invention provides a novel process of synthesizing curcuminoids without using carriers/emulsifiers of higher strength than available grades in market to minimize the dose/quantity and make more bioavailable.

According to an embodiment of the present invention, a composition containing curcumin as the main ingredient in which the bioavailability of curcumin is high is provided.

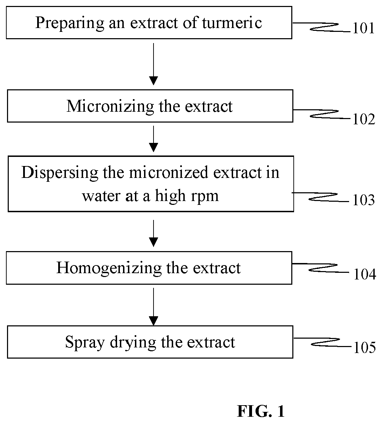

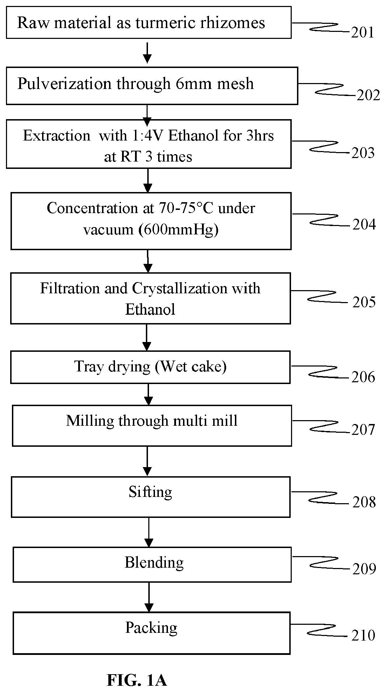

**FIG. 1** is a flow chart showing the steps involved in the method of synthesizing a turmeric based composition, according to an embodiment of the present invention. With respect to **FIG. 1,** a method of synthesizing a turmeric based composition comprising preparing an extract of turmeric (**101**), micronizing the extract (**102**), dispersing the micronized extract in water at a high rpm (**103**), homogenizing the extract (**104**), and spray drying the extract (**105**). The turmeric extract is 95%. The micronization is performed at an input pressure of 1 to 7 bars and an injector pressure of 0.2 to 0.5 bar using a mcironizer machine. The micronization is performed at a loading rate of 0.1 to 5Kg/hr keeping gas flow rate of 30 to 100 m/hr of the micronizer machine. The dispersion is done in water at 5000 to 7500 rpm using high shear blades at hot condition temperature of 70-80°C. The extract is spray dried at an inlet temperature of 190-200°C and outlet temperature of 90-100°C at a feed rate of 60L/hr.

According to an embodiment of the present invention, a novel water dispersible composition containing curcumin having enhanced bioavailability is prepared by micronization of the 95% of turmeric extract followed by dispersion in water, homogenization and spray drying.

**Preparation of Turmeric extract 95%:**

The extract of turmeric is prepared by grinding the turmeric into powder. After grinding, the method includes the step of treating the turmeric powder with ethanol, during which, the residue is extracted. **FIG. 1A** is a flow chart showing the steps involved in the process of preparing an extract of turmeric, according to an embodiment of the present invention. With respect to **FIG. 1A,** the raw material which turmeric rhizome are taken (**201**) and pulverized through a 6 mm mesh (**202**). The pulverized turmeric is extracted with 1.4V ethanol for 3 hours at RT 3 times (**203**). The extract is concentrated at 70-75°C under vacuum (600mmHg) (**204**). The extract is filtered (**205**) and dried using a tray drying (wet cake) method (**206**). The extract is milled through a multimill (**207**), sifted (**208**), blended (**209**) and packed (**210**).Then, the extract is micronized.

**Micronization:** The Micronization is carried out in a micronizer machine. The Micronization is carried out at an input pressure of 1 to 7 bars, injector pressure of 0.2 to 0.5 bar at a loading rate of 0.1 to 5Kg/hr keeping gas flow rate of 30 to 100m/hr. The micronization is done for at least 20 hours for a batch size of 100 kg. The micronized extract is dispersed in water.

**Dispersion:** The extract is dispersed in water at 5000 to 7500rpm using high shear blades at hot condition temperature of 70-80°C.

**Homogenization:** In this step the particles of the turmeric are made uniformly distributed throughout a fluid. Homogenization carried out at 60-65°C and pressure 150-175/bar using high pressure homogenizer.

**Spray drying:** The extract is spray dried after the dispersion step. The extract is spray dried at an inlet temperature of 190-200°C and outlet temperature of 90-100°Cat a feed rate of 60L/hr. The total solids of input solution are 15 to 20%w/v.

According to an embodiment of the present invention, the particle size of the turmeric ranges between 0.150 (100 mesh) to 0.250 mm (60 mesh).

It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the claims presented in the complete specification or non-provisional application.

**EXPERIMENTAL DETAILS**

The composition according to the present invention was synthesized as per the process of the present invention. Table 1 below shows the composition. In the specification, the terms "Turmimax®" and the "turmeric composition" of the present invention are used interchangeably. Further, the term "Turmimax®" represents the turmeric composition of the present invention.

TABLE 1: Composition of Tumimax® / composition of turmeric of present invention

| INGREDIENT(S) COMPOSITION | | | |
|---|---|---|---|
| ACTIVES | | | |
| COMMON OR USUAL NAME | SYNONYM | ORIGIN | WEIGHT PERCENT |
| Turmeric extract 95% | Turmeric extract | India | 100% |
| NO EXCIPIENTS/CARRIERS/ADDITIVES USED | | | |
| - | - | - | - |

The extract of turmeric was prepared by grinding the turmeric into powder. The turmeric rhizome were taken and pulverized through a 6 mm mesh. The pulverized turmeric was extracted with 1.4V ethanol for 3 hours at RT 3 times. The extract was concentrated at 70-75°C under vacuum (600mmHg). The extract was filtered and dried using a tray drying (wet cake) method. The extract was milled through a multimill, sifted, blended and packed. Then, the extract was micronized. Table 2 below shows the parameters of carrying out the Micronization.

TABLE 2: Parameters of Micronization step

| Instruments | Micronizer |
|---|---|
| Input pressure | 1 to 7 bars |
| Injector pressure | 0.2 to 0.5bar |
| Loading rate | 0.1 to 5Kg/hr |
| Gas flow | 30 to 100m/hr |
| Time | Depends on batch size |

The extract was dispersed in water at 5000 to 7500 rpm using high shear blades at hot condition temperature of 70-80°C. The dispersion was done using a spray drying machine. The inlet temperature was kept at 190-200°C with a feed rate of 60L/hr. The total solids of input solutionwas15 to 20%w/v. Table 3 below shows the parameters of carrying out the dispersion.

TABLE 3: Parameters of dispersion step

| Instruments | Spray drying |
|---|---|
| Inlet temperature | 190-200°C |
| Outlet temperature | 90-100°C |
| Feed rate | 60L/hr |
| Total solids of input solution | 15 to 20%w/v |

Bioavailability

The studies were carried out using male wistar albino rats (180-200g). They were randomly divided into 2 groups with six rats(Group I and II) in each group. Rats were fasted overnight for 12 h, with free access to water and body weight was measured before dosing of the animals. Group I and II were fed with test dose orally at a dose volume of 10ml/kg body weight. At different time intervals of 0, 0.5, 1, 2, 4, 8, 12 and 24 hr rats were anaesthetized under isoflurane anesthesia and blood samples collected by retro-orbital route of 500μL in 1 ml centrifuge tubes containing EDTA. The blood samples were centrifuged at 4000 rpm for 10 minutes at 4°C within 1hr of collection. After centrifugation, plasma samples were separated and taken for HPLC analysis. 500μl of rat plasma was transferred into a centrifuge tube, 1ml of ethanol was added, and centrifuged at 5000rpm for 30minunder -4°C. The supernatant liquid was decanted and dried under nitrogen environment.500μl of methanol was added and transferred into a HPLC vial.

HPLC-UV analysis was performed on a Shimadzu LC-10AT (Shimadzu Corporation, Kyoto, Japan) instrument equipped with an UV-Vis detector and a 4.6 mm x 25 cm, C18 column. Flow rate 0.8 ml per minute. 20μl of diluent was injectedinto the chromatograph as a blank run. Then 20μl each of the standard and sample were injected and the peaks in the chromatograph was recorded and analysed at 420 nm. The retention time of Curcuminoids in standard and sample was the same. The relative standard deviation from replicate injections of standard preparation was not more than 2.0 %.

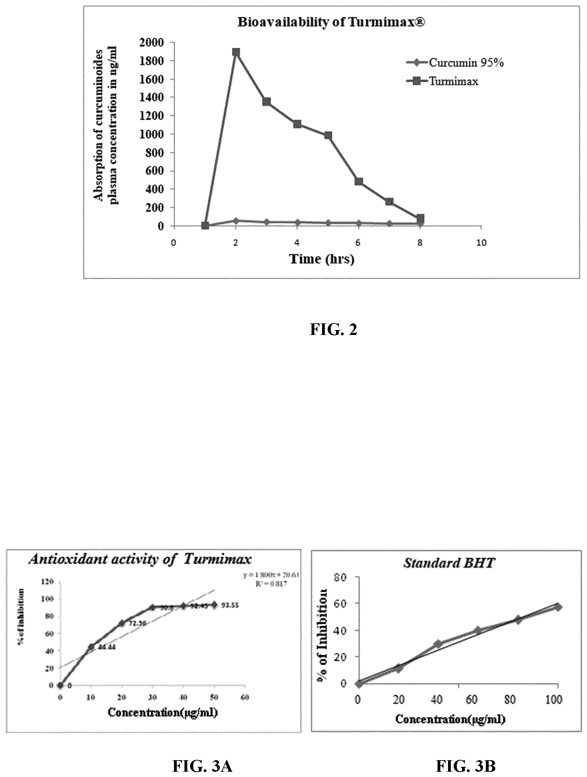

The bioavailability of the composition of curcuminoids (Turmimax®) is shown in **FIG. 2. FIG. 2** shows the plasma concentration of curcuminoids after application of a single oral dose(500mg/kg.b.w) of Turmimax® in rats, according to an embodiment of the present invention. Each point represents mean ± S.D. (n=6). With respect to **FIG. 2,** the composition has significantly increased more absorption and higher bioavailability compared to curcumin 95%. The bioavailability of the compositionwas found to be 1890ng/ml as compared to curcumin 95% found 54.62ng/ml (35X). It is clearly showed the presence of curcuminoids in higher concentration in the serum at 30 min of administration of the composition. With the availability of highly bioavailable forms of the present composition that have >35-fold better absorption than normal curcumin, appropriate human pharmacological studies will be conducted, knowing that desirable blood levels of the active forms of the present composition are achievable.

Antioxidant activity by DPPH (2, 2-diphenyl 1, 1-picrylhydrazyl) radical scavenging assay)

Turmimax® or the turmeric composition of the present invention is manufactured and registered by Star Hi Herbs Pvt. Ltd, Jigani, Bangalore, Karnataka, India. The antioxidant screening of the sample was measured according to the Blois method by using UV- visible spectrophotometer at 517nm [1]. The 0.1mm DPPH radical were prepared in methanol and 1 ml of this solution were mixed with 3 ml of sample at different concentrations ranging from 10, 20, 30,40 and 50μl/ml respectively. The control was maintained with 1 ml of DPPH solution and 3mL of methanol without sample. The reaction mixtures were incubated at ambient temperature for 30 minutes. The absorbance was measured at 517 nm against the blank solution. Butylated hydroxytoluene (BHT) with different concentrations (20, 40, 60, 80 and 100μg/ml) was used as positive control. The radical scavenging activity of DPPH was calculated as mentioned below.

$$\% \text{ Inhibition} = (\text{Control ABS} - \text{Sample ABS})/ \text{Control ABS} \times 100.$$

DPPH free radical scavenging assay Free radical scavenging activity of Turmimax® and BHT were tested using the DPPH method and the results are shown in **FIG. 3.** In this study, the free radical scavenging ability of each sample was evaluated through recording the change of absorbance produced by the reduction of DPPH. **FIG. 3** showing the DPPH free radical scavenging activity (IC50μg/ml) of Turmimax®/composition of the present invention, according to an embodiment of the present invention. With respect to **FIG. 3,** the IC50 was found to be 16.31 ± 0.06 μg/ml for Turmimax® and for standard BHT, it was found to be 82.65 ± 0.02 μg/ml. The results obtained indicated that higher percentage of DPPH scavenging activity may be attributed to the high reducing power and higher total phenolic contents present in Turmimax®. In DPPH assay, the antioxidants were able to reduce DPPH to yellow colored diphenyl picrylhydrazone (Frankel and Meyer, 2000). The method based on the reduction DPPH in alcoholic solution in the presence of hydrogen donating antioxidant due to formation of the non-radical form DPPH-H in the reaction. DPPH is usually used as a reagent to evaluate free radical and accepts an electron or hydrogen radical to become a stable diamagnetic molecule (Oyaizu, 1986). Table 4 shows the results of the DPPH scavenging activity.

**Table-4 Results of DPPH scavenging activity**

| Sl.No. | Turmimax conc (μg/ml) | OD at 517 nm | % Inhibition | IC$_{50}$μg/ml |
|---|---|---|---|---|
| 1 | Control | 0.729 | 0 | 16.31 |
| 2 | 10 | 0.405 | 44.44 | |
| 3 | 20 | 0.200 | 72.56 | |
| 4 | 30 | 0.067 | 90.80 | |
| 5 | 40 | 0.055 | 92.45 | |
| 6 | 50 | 0.047 | 93.55 | |

**Hydrogen peroxide scavenging assay**

Hydrogen peroxide scavenging potential of curcumin was determined using the method described by Jayaprakasha et al. (2004). A solution of hydrogen peroxide (20 mM) was prepared in phosphate buffer saline (PBS, pH 7.4). Different concentrations of Turmimax®/the composition of the present invention (20 to 80 μg/ml) in ethanol (1 ml) were added to 2 ml of hydrogen peroxide solution in PBS. After 10 min, the absorbance was measured at 230 nm using spectrophotometer (Shimadzu UV1800) against a blank solution that contained hydrogen peroxide solution without Turmimax. All the test was performed in Triplicate. The % Scavenging activity was measured by using formula.

$$\% \text{ scavenged } [H_2O_2] = [(A0-A1)/A0] \times 100$$

The ability of Turmimax® to scavenge hydrogen peroxide is shown in Table-5 and compared with that of ascorbic acid as reference compound. The IC50 was found to be 39.45µg/ml for Turmimax and for standard ascorbic acid, it was found to be 47.20 µg/ml **(FIG-4A and FIG.4B)**. **FIG-4A** shows the Hydrogen peroxide scavenging activity (IC50µg/ml) of Turmimax®/composition of the present invention, and **FIG. 4B** shows the antioxidant activity of the ascorbic acid, according to an embodiment of the present invention. Biological systems can produce hydrogen peroxide. Hydrogen peroxide can be formed in vivo by several oxidizing enzymes such as superoxide dismutase. Turmimax® has effective hydrogen peroxide scavenging activity. It is known that H2O2 is toxic and induces cell death in vitro (Aoshima et al., 2004). Hydrogen peroxide can attack many cellular energy-producing systems. For instance, it deactivates the glycolytic enzyme glyceraldehyde-3- phosphate dehydrogenase (Hyslop et al., 1988). The present investigation indicates that Turmimax® shows promising properties for antioxidant. The scavenging activity of these Turmimax® were determined by using hydrogen peroxide assay. Results indicated that the Turmimax® possess higher scavenging activity than BHT. The study serves as a scientific data for the use of Turmimax® exhibited significant activity of antioxidant drug.

**Table 5: Results of Hydrogen Peroxide scavenging activity**

| Sl.No. | Turmimax conc (µg/ml) | % Inhibition | IC$_{50}$µg/ml |
|--------|------------------------|--------------|----------------|
| 1 | 20 | 40.90±2.27 | **39.45** |
| 2 | 40 | 59.59±1.15 | |
| 3 | 60 | 70.49±2.21 | |
| 4 | 80 | 81.06±2.27 | |

**Anti-inflammatory activity of Turmimax® by Croton oil-Induced Ear Edema method.**

The studies were carried out using 30 Swiss albino mice and male wistar albino rats. This experimental procedure was performed using the method of Hosseinzadeh et al. Swiss Albino mice of either sex weighing 25-35 grams were divided into five groups of six animals each. The dosage of the drugs administered to the different groups was as follows. Group I Control (normal saline 10 ml/kg), Group II Negative control (2.5% Croton oil), Group III diclofenac sodium (10mg/kg) was used as a standard drug, IV Turmimax® 100 mg/kg and Group- V Turmimax® 200 mg/kg b.wt. The edema was induced in each mouse by applying 50 µL croton oil to the inner surface of the right ear. Ninety minutes after croton oil daubing, the mice were executed by cervical dislocation, and both ears were removed and weighed.

The topical application of croton oil caused an increase in the weight of the ears of the animals due to the development of edema, as can be observed in the control group (as shown in **FIG. 5** and Table-6). **FIG. 5** shows the anti-inflammatory activity of Turmimax®/composition of the present invention on croton oil induced ear edema in mice, according to an embodiment of the present invention. The edema was induced in each mouse by applying 50 µL croton oil to the inner surface of the right ear. Ninety minutes after croton oil daubing, the mice were executed by cervical dislocation, and both ears were removed and weighed. The Turmimax® reduced the edema significantly when compared to the control (P < 0.05 and P < 0.01, respectively). Diclofenac sodium10mg/kg body weight used as a standard anti-inflammatory drug. Turmimax® (100 and 200 mg/kg) showed a percentage of inhibition edema reduction of 44.65% (P< 0.5) and 57.25% (P< 0.01) respectively.

**Table-6. Effects of Turmimax®, control and diclofenac sodium (Diclofenac 10 mg/kg) on Croton oil induced ear edema**

| Treatment | | | | % inhibition | |
|-----------|------|------|------|--------------|--------|
| | 0hr | 2hr | 4hr | At 2hr | At 4hr |
| Control | 0.306±0.091 | 0.306±0.019 | 0.306±0.069 | - | |
| Croton oil | 0.308±0.023 | 0.786±0.039 | 0.786±0.059 | - | |

(continued)

| Treatment | | | | % inhibition | |
| --- | --- | --- | --- | --- | --- |
| | 0hr | 2hr | 4hr | At 2hr | At 4hr |
| Standard (Diclofenac sodium 10m g/kg p.o) | 0.310±0.051 | 0.320±0.029** | 0.326±0.019** | 59.28% | 58.52% |
| Low dose (100mg/kg p.o) | 0.310±0.032 | 0.425±0.091* | 0.435±0.039* | 45.92% | 44.65% |
| High dose (200mg/kg p.o) | 0.305±0.09 | 0.325±0.079** | 0.335±0.029** | 58.65% | 57.25% |
| All values are expressed as mean ± SD; **= P < 0.01, *= P < 0.05 v/s croton oil control | | | | | |

**Anti-inflammatory activity of Turmimax® by Histamine-Induced Inflammation**

Wister albino rats of either sex weighing 180- 200 grams were divided into five groups of six animals each. The dosage of the drugs administered to the different groups was as follows. Group -I Control (normal saline 10 ml/kg), Group - II Negative control (histamine), Group III Diclofenac sodium (10mg/kg), IV Turmimax® 100 mg/kg and Group V - Turmimax® 200mg/kg. b.wt. Diclofenac sodium served as the reference standard anti-inflammatory drug. Turmimax® were administered to rats at 1 hr before the induction of inflammation. Edema was assessed as the difference in paw volume between the control and 0.5, 1, 2, 3, and 4 h after the administration of the inflammatory agent inhibition.

Inflammation was induced in rats by the injection of 0.1 mL 0.1% histamine in normal saline into the subplantar tissue of the right hind paw in rats. Test drugs were administered to rats at 1 hr before the induction of inflammation. Control group received 10 mL/kg body weight of distilled water orally. Edema was assessed as the difference in paw volume between the control and 0.5hr, 1hr, 2hr and 4hr after the administration of the inflammatory agent, inhibition. The results were given in the following Table-7 and **FIG. 6. FIG. 6** shows the anti-inflammatory activity of Turmimax®/composition on histamine-induced paw edema in rats, according to an embodiment of the present invention.

**Table-7. Effect of Turmimax®, control and diclofenac sodium (Diclofenac 10 mg/kg) on Histamine induced paw edema**

| Treatment | 0 hr | 0.5hr | 1hr | 2hr | 4hr |
| --- | --- | --- | --- | --- | --- |
| Group-I (control-only vehicle) | 1.94±0.3 | 1.9±0.9 | 1.9±0.9 | 1.9±0.9 | 1.9±0.9 |
| Group-II (Toxic control) vehicle + histamine | 1.9±0.6 | 2.6±0.2 | 3.2±0.4 | 3.8±1.4 | 3.8±1.4 |
| Group-III (Diclofenac sodium10mg/kg p.o + histamine) | 1.9±0.7 | 2.3±0.9±0.4** | 2.7±0.1** | 2.2±0.6** | 2.2±0.2** |
| Group-IV (Low dose (100mg/kg p.o) + histamine) | 1.9±0.6 | 2.6±0.5* | 3.0±1.2* | 2.9±0.9* | 2.8±0.7* |
| Group-V (High dose (200mg/kg p.o) + histamine) | 1.9±0.8 | 2.1±0.6** | 2.8±0.8** | 2.3±0.6** | 2.3±0.2** |
| All values are expressed as mean ± SD; **= P < 0.01 *= P < 0.05 v/s Histamine control | | | | | |

**Anti-Cancer Properties of Turmimax® In Human Cervical Adenocarcinoma by MTT Assay**

HeLa cells were seeded in a 96-well plate (Corning, USA) at a density of 20 000 cells per well. Cells were treated with concentrations of Turmimax®ranging from 0 - 100 μg/ml for a duration of 24 h. The media was replaced prior to preparation of MTT Assay where MTT reagent (5 mg/ml; Cat. No. 4060, Himedia) was added to each well at a final concentration of 0.5 mg/ml. The plate was incubated for 3 h at 37°C. The MTT reagent medium was replaced by DMSO to solubilize the MTT formazan crystals by gentle rocking on a gyrator. Cytotoxicity of Turmimax® in HeLa cells was evaluated through MTT assay activity at concentrations of 6.25, 12.5, 25, 50 and 100 $\mu$g/ml after 24h exposure period. Cell proliferation was shown to decrease with increasing concentrations of Turmimax® at both microscopic observations (**FIG. 7A**) and MTT absorbance rates (**FIG. 7B**). **FIG. 7A** shows the morphological changes to different concentrations of Turmimax® for 24 hrs in Hela cells and **FIG. 7B** shows cytotoxicity effect of Turmimax® against HeLa cell line after 24 h incubation. With respect to **FIG. 7A,** (i) shows for 6.25$\mu$g/ml, (ii) shows for 12.5 $\mu$g/ml, (iii) shows for 25 $\mu$g/ml, (iv) shows for 50 $\mu$g/ml, and (v) shows for 100 $\mu$g/ml of tumimax concentration. MTT analyses have shown that Turmimax® may possess potentially significant cytotoxic effects on HeLa cells baring an $IC_{50}$ value of approximately 87.89 $\mu$g/ml.

**Flow Cytometry**

Cells were seeded at a density of 3 x $10^5$ cells/2 ml in a 6-well culture plate (Biolite, Thermo) and incubated overnight at 37°C. Each well was treated with the appropriate concentration of the apoptotic standard, campothecin or Turmimax® in fresh 2 ml DMEM medium for 24 h. Post-treatment cells were washed with PBS (Cat No. TL1006, Himedia) and harvested. Cells were centrifuged for 5 minutes at 300 xg at room temperature and the supernatant was carefully decanted. The pellet was washed twice with PBS prior to staining with 5 $\mu$l of Annexin V FITC (Cat No: 51-65874X, BD Biosciences). Each were vortexed and incubated in the dark at room temperature for 15 minutes. Cells were stained with 5 $\mu$l Propidium Iodide (PI; Cat No. 51-66211E, BD Biosciences) and 400 $\mu$l of 1 x Annexin V binding buffer. Cells were analysed for apoptotic activity in a BD FACS Calibur (BD Biosciences).

**Cell cycle arrest:** Cells were cultured at a density of 2 x $10^5$ cells/2 ml and incubated at 37 °C for 24h. Cells were replenished with fresh media and treated with Turmimax® or camptothecin. Cells were harvested and centrifuged for 5 minutes at 300 xg. Cells were rinsed with PBS and fixed in 1 ml of cold 70 % ethanol on ice for 30 minutes. Cells were centrifuged and washed twice with PBS. After the wash step, cells were stained with 400 $\mu$l PI/RNAse staining solution (Cat No: 550825, Sigma) and incubated for 20 minutes at room temperature. Using the BD FACS Calibur flow cytometer, cells were analysed for cell cycle arrested.

**Turmimax® apoptosis induction in HeLa cells: Turmimax® apoptosis** induction activity was evaluated using the $IC_{50}$ value of 87.89 $\mu$g/ml on HeLa cells. The ability of Turmimax® to induce apoptosis in cervical adenocarcinoma cells was in comparison to the apoptotic standard, camptothecin. **FIG. 8** shows the Apoptosis Induction of Turmimax® in HeLa cells, according to an embodiment of the present invention. With respect to **FIG. 8,** A is for HeLa cell control, B is for HeLa cell + STD, C is for HeLa cell + Turmimax and D shows the graph. Cells were treated with 87.89 $\mu$g/ml of Turmimax® and camptothecin (apoptotic standard) for 48h. Flow cytometric analysis for necrotic (PI) and apoptotic (Annexin V-FITC) positive cell populations. There is a clear distinction of apoptotic activity in comparison to the untreated (control) cells (**FIG. 8**) with the highest percentage of viable cells while Turmimax® had a significantly larger apoptotic cell population to camptothecin, 82.93 % to 44.19 % respectively. The observed necrotic (dead) cell population per treatment was relatively the same with a clear absence of dead cells in the control treatment. This clearly indicates that the turmeric rhizome extract can elicit anticancer effects through the apoptotic pathway in HeLa cells.

**G0/G1 and S phase cell cycle arrest in HeLa cells by Turmimax®:** HeLa cell were once again treated with the $IC_{50}$ concentration to evaluate Turmimax® cell cycle arrest activity. **FIG. 9** shows the Cell Cycle Analysis of Turmimax® against HeLa cells using BD FACS Caliber, according to an embodiment of the present invention. With respect to **FIG. 9,** A is for HeLa cell control, B is for HeLa cell + STD, C is for HeLa cell + Turmimax and D shows the graph. PI histogram of the gated Cell singlets distinguishes cells at the Sub GO/G1, GO/G1, S, and G2/M cycle phases. In comparison to the control (untreated), there was a decrease in the percentage of cells arrested at the GO/G1 and G2/M phases but an increase in the sub GO/G1 and S phases (**FIG. 9**) in the Turmimax® treatment. Camptothecin treatment at 10 $\mu$M showed a drastic reduction in cell cycle arrest in the GO/G1 phase population and increased observed percentage in the G2/M phase however both these phases had the highest cell cycle arrested populations within the treatment. Turmimax® clearly shows promising interaction with HeLa cells to induce apoptosis through cell cycle arrest. Turmimax® at an $IC_{50}$ value of 87.89$\mu$g/ml shows promising properties as being a potential anti-cancer therapeutic agent in human cervical adenocarcinomas and possibly other cancer types.

**In vitro Anti-Inflammatory Activity**

Raw 264.7 and HepG2 cell lines grown in DMEM high glucose medium supplemented with 10% FBS, 100$\mu$l of Antibiotic-Antimycotic (100X) in a $CO_2$ incubator at 37°C with 20% oxygen and 5% $CO_2$ in saturated humidity. Post 24hr seeding event, medium was changed prior to treatment with Turmimax® and $H_2O_2$ or LPS. Turmimax® and $H_2O_2$/LPS was dissolved in DMSO and vortexed to ensure 100% solubility. The final concentration of DMSO added to the cell culture medium was below 0.1%. The final Turmimax® concentrations added to the cell culture medium ranging from 6.25-100$\mu$g/ml respectively. A 0.1% DMSO concentration was used as a Vehicle control which does not exhibit any cytotoxicity on both the cells.

**MTT Assay**

An MTT assay was performed to evaluate the HepG2 and Raw 264.7 cell viability. The MTT cytotoxicity study of Turmimax® on human liver and murine cells lines were evaluated by colorimetry. Various concentrations were used to evaluate the toxicity of Turmimax® and the $IC_{50}$ concentration was calculated from the resultant dose-response curve. The relative Turmimax® cytotoxicity on Raw 264.7 cell lines are depicted in Table-8. **FIG. 10** shows the percentage viability of HepG2 and Raw 264.7 cells treated with various concentrations of Turmimax® for 24 hrs as determined by MTT assay, according to an embodiment of the present invention. Turmimax® exhibited no cytotoxic effect on Raw 264.7 cells at concentrations of and below 50μg/ml. On Raw 264.7 cell line, Turmimax® exhibited cytotoxicity at a concentration of 100μg/ml. Therefore, the non-cytotoxic concentration of 50μg/ml was selected for Raw 264.7 cell lines for further mechanistic studies.

**Table. 8 % of cell viability values Turmimax® towards HepG2 and Raw 264.7 cells at 24hrs as determined by MTT assay.**

| Cell line/ Concentration | 0 | 6.25 | 12.5 | 25 | 50 | 100 |
|---|---|---|---|---|---|---|
| HepG2 | 100 | 87.32246 | 91.21515 | 94.79221 | 98.79011 | 99.36875 |
| Raw 264.7 | 100 | 97.56543 | 94.09617 | 90.44431 | 79.18442 | 63.42057 |

For the evaluation of anti-inflammatory activity, Raw 264.7 cells were cultured in a 6 well plate and treated with 3 different culture conditions viz., LPS (2ug/ml), LPS (2μg/ml)+ Turmimax® (50μg/ml) and Untreated without any treatment. Briefly the cells were pre stimulated with 2 μg/ml of LPS for 3 hours to induce inflammation and following stimulation, the cells were either treated with 50μg/ml of Turmimax® or LPS stimulated alone (negative control) with 2 ml DMEM medium. Cells were incubated for 24 hrs and harvested into centrifuge tubes (BD Biosciences) and centrifuged at 300 x g for five minutes in a Remi: R-8°C centrifuge and were washed twice with D-PBS. The pelleted cells were incubated at room temperature with 0.5 ml BD Cytofix/Cytoperm for 10 minutes and washed with 0.5% Bovine Serum Albumin solution (1x PBS and 0.1% sodium azide). Cells were incubated with 20μl of PE-Mouse Anti Human interleukin 8 (IL-8) antibody/Anti Human IL-12 separately for 30 minutes in the dark at 25°C and expression measured using a BD FACS Calibur flow cytometer (BD Biosciences) and data analyzed by Cell Quest Pro software version 6.

**Statistical Analysis:** All the data were analyzed using Microsoft Excel 2007 version in creating graphical representation of the mean with calculated standard errors. Flow Cytometric data was analyzed using Cell Quest Pro software version 6.Turmimax® exhibits significant anti-inflammatory effect through IL-8 and IL-12 inhibition in LPS stimulated macrophage cells.LPS-induced inflammation, in *in vitro* cell lines and in animals, represents a standard paradigm for studying inflammation. IL-8 and IL-12 are the pro-inflammatory cytokines that are expressed drastically in cells exhibit more inflammation. In the current study, the ability of Turmimax®to elicit anti-inflammatory effects on RAW 264.7 cells by evaluating pro-inflammatory cytokines, IL-8 and IL-12, expression. **FIG. 11** shows the Flow cytometry histograms of mean pro-inflammatory cytokines (IL-8 & IL-12) in Raw 264.7 cells pre-stimulated with LPS followed by 24 hrs exposure to Turmimax®, according to an embodiment of the present invention. With respect to FIG. 11, A is for RAW 264 cell control, B is for RAW 264 LPS, C is for RAW 264 + Turmimax, D is for RAW 264 Cell control, E is for RAW 264 LPS, and F is for RAW 264 + Turmimax, G shows the graph. LPS served as a positive control (n=3, mean ± standard error). In the untreated cells, the relative interleukin expression is very low for both cytokines (**FIG. 11**). On the other hand, LPS stimulated cells alone, exhibited 5-6 times higher expression than the untreated group. However, the Turmimax® treated cells following LPS stimulation were expressing lower expressions than the LPS alone treated cells. *In vitro* studies have shown that curcumin inhibits inflammation in mouse fibroblast cell line [13, 16]. Turmimax® showed a substantial inhibitory effect on pro-inflammatory cytokine production, IL-8 and IL-12. Curcumin has been shown to inhibit macrophage-derived cytokines like IL-8, monocyte inflammatory protein-1 and tumor necrosis factor a [13]. At a concentration of 20 μM, curcumin showed pro-inflammatory cytokine inhibition of IL-8 in adult peripheral mononuclear monocyte cells and preterm lung inflammatory cells and IL-12 thereby preventing allergic encephalomyelitis in T-lymphocytes [12]. Kerotinocytes treated with 6.7 μg/ml curcumin showed attenuated levels of IL-8 and the researchers believed that it could be used as a short-term treatment in type 2 diabetic nephropathy [5]. A study by [16] showed in LPS stimulated mouse splenocytes there was potent inhibitory release of IL-12 when treated with 0.8 to 100 μg/ml of curcumin extract. Human phase one trial has shown patients and animal models administered with a daily curcumin intake between 1125-8000 mg/day showed no cytotoxic effect and exhibited anti-inflammatory effects through various target molecules including IL-12 [2]. Thus Turmimax® inhibited the expression of pro-inflammatory cytokines in LPS stimulated macrophages.

In conclusion, Turmimax® possesses biological actions in cell proliferation, suppression of ROS expression in $H_2O_2$ treated HepG2 cells and inhibiting the expression of pro-inflammatory cytokines in LPS stimulated murine macrophages. This study's results significantly indicated that Turmimax® is a potent and promising natural compound for the treatment of liver and inflammatory related diseases. The current work innovatively illustrated the anti-inflammatory expression studies through flow cytometric analyses.

## ADVANTAGES OF INVENTION

The present invention provides a water dispersible solution of turmeric which overcomes the drawback of poor bioavailability exhibited by currently available curcumin compositions. The major problems of poor bioavailability of curcumin are poor absorption, rapid metabolism and rapid systemic elimination. Hence the present invention provides a composition of curcumin that enhances the absorption of curcumin in blood. Such composition may be further used as a natural cure for treatment of many diseases like arthritis, gastrointestinal disorder, cancer, etc.

The present invention provides a novel process to produce curcuminoids without carriers/emulsifiers of higher strength thanavailable grades of water dispersiblein market to minimize the dose/quantity and make more bioavailable which can be used in instant bevarages, shots, gummies and soft gels.

According to an embodiment of the present invention, a novel water dispersible curcumin having enhanced bioavailability is provided. The composition is used for making of effervescent beverages, gummies, soft gels, tablets, capsule, shots, drinks.

It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the claims presented in the complete specification or non-provisional application.

## Claims

1. A method of synthesizing a turmeric based composition having enhanced bioavailability, comprising:

    preparing an extract of turmeric;
    micronizing the extract;
    dispersing the micronized extract in water at a high rpm;
    homogenizing the extract; and
    spray drying the extract.

2. The method as claimed in claim 1, wherein the turmeric extract is 95%.

3. The method as claimed in claim 1, wherein the micronization is performed at an input pressure of 1 to 7 bars and an injector pressure of 0.2 to 0.5bar using a mcironizer machine.

4. The method as claimed in claim 1, wherein the micronization is performed at a loading rate of 0.1 to 5Kg/hr keeping gas flow rate of 30 to 100m/hr of the micronizer machine.

5. The method as claimed in claim 1, wherein the dispersion is done in water at 5000 to 7500 rpm using high shear blades at hot condition temperature of 70-80°C.

6. The method as claimed in claim 1, wherein the extract is spray dried at an inlet temperature of 190-200°C and outlet temperature of 90-100°C at a feed rate of 60L/hr.

7. The method as claimed in claim 1, wherein the micronization is done for 20 hour for a batch of 100 kg.

8. The method as claimed in claim 1, wherein the turmeric based composition has an enhanced bioavailability, antioxidant, antiproliferative and/or anti-inflammatory useful for treatment of cancer and inflammation.

9. The method as claimed in claim 1, wherein the turmeric based composition has a therapeutically sufficient amount of the water dispersible curcumin complex of turmeric as active ingredients and a pharmaceutically acceptable vehicle or carrier.

10. The method as claimed in claim 9, wherein the therapeutically sufficient amount is between 0.01 mg and 1000 mg per kilogram of body weight of the individual to which it is administered.

11. The method as claimed in claim 1, wherein turmeric based composition is having enhanced bioavailability useful for making effervescent beverages, gummies, soft gels, tablets, capsule, shots, drinks.

Preparing an extract of turmeric — 101

↓

Micronizing the extract — 102

↓

Dispersing the micronized extract in water at a high rpm — 103

↓

Homogenizing the extract — 104

↓

Spray drying the extract — 105

**FIG. 1**

Raw material as turmeric rhizomes — 201

Pulverization through 6mm mesh — 202

Extraction with 1:4V Ethanol for 3hrs at RT 3 times — 203

Concentration at 70-75°C under vacuum (600mmHg) — 204

Filtration and Crystallization with Ethanol — 205

Tray drying (Wet cake) — 206

Milling through multi mill — 207

Sifting — 208

Blending — 209

Packing — 210

**FIG. 1A**

**FIG. 2**

**FIG. 3A**                    **FIG. 3B**

FIG. 4A

FIG. 4B

FIG. 5

FIG.6

(i)    (ii)    (iii)    (iv)    (v)

**FIG. 7A**

**FIG. 7B**

**FIG. 8**

**FIG. 9**

**FIG. 10**

**FIG. 11**

Europäisches
Patentamt
European
Patent Office
Office européen
des brevets

# EUROPEAN SEARCH REPORT

Application Number

EP 21 18 6828

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 400 932 A1 (MCEPHARMA S R O [CZ]; IMCOPHARMA A S [CZ]) 14 November 2018 (2018-11-14) | 1,2,7-11 | INV. A61K36/9066 B01D11/00 |
| Y | * paragraphs [0003], [0006], [0007], [0011], [0014], [0043], [0046] * * claim 1 * | 1-11 | |
| Y | US 2016/089343 A1 (NATHAN CHERIE-ANN [US]) 31 March 2016 (2016-03-31) * paragraphs [0015], [0018] * | 1-11 | |
| Y | CN 108 404 040 A (LI JUN) 17 August 2018 (2018-08-17) * the whole document * | 1-11 | |
| Y | MARTINS RODRIGO MOLINA ET AL: "Curcuminoid content and antioxidant activity in spray dried microparticles containing turmeric extract", FOOD RESEARCH INTERNATIONAL, ELSEVIER, AMSTERDAM, NL, vol. 50, no. 2, 30 June 2011 (2011-06-30), pages 657-663, XP028985411, ISSN: 0963-9969, DOI: 10.1016/J.FOODRES.2011.06.030 * the whole document * | 1-11 | |

TECHNICAL FIELDS
SEARCHED (IPC)

A61K
B01D

The present search report has been drawn up for all claims

| Place of search | Date of completion of the search | Examiner |
|---|---|---|
| Munich | 16 September 2021 | Weisser, Dagmar |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 3 943 096 A1**

**ANNEX TO THE EUROPEAN SEARCH REPORT
ON EUROPEAN PATENT APPLICATION NO.**

EP 21 18 6828

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2021

| Patent document cited in search report | | Publication date | Patent family member(s) | | Publication date |
|---|---|---|---|---|---|
| EP 3400932 | A1 | 14-11-2018 | CA | 3004220 A1 | 08-11-2018 |
| | | | CZ | 307916 B6 | 21-08-2019 |
| | | | EP | 3400932 A1 | 14-11-2018 |
| | | | US | 2018325842 A1 | 15-11-2018 |
| US 2016089343 | A1 | 31-03-2016 | EP | 3200777 A1 | 09-08-2017 |
| | | | US | 2016089343 A1 | 31-03-2016 |
| | | | US | 2016374962 A1 | 29-12-2016 |
| | | | WO | 2016053903 A1 | 07-04-2016 |
| CN 108404040 | A | 17-08-2018 | NONE | | |

EPO FORM P0459

For more details about this annex : see Official Journal of the European Patent Office, No. 12/82